# EUROPEAN PATENT SPECIFICATION

(11) **EP 4 208 133 B1**
(45) Date of publication and mention of the grant of the patent: **12.06.2024**
(21) Application number: 21783092.6
(22) Date of filing: 30.08.2021
(51) Int. Cl.: A61F 5/44, A61F 5/453, A61F 5/455

(54) **FLUID COLLECTION DEVICES AND SYSTEMS HAVING ONE OR MORE SECUREMENT STRAPS, AND METHODS OF USE**
FLÜSSIGKEITSSAMMELVORRICHTUNGEN UND -SYSTEME MIT EINEM ODER MEHREREN BEFESTIGUNGSBÄNDERN UND VERFAHREN ZUR VERWENDUNG
DISPOSITIFS ET SYSTÈMES DE COLLECTE DE FLUIDE AYANT UNE OU PLUSIEURS SANGLES DE FIXATION, ET PROCÉDÉS D'UTILISATION

(30) Priority: 02.09.2020 US 202063073553 P
(43) Date of publication of application: 12.07.2023
(73) Proprietor: Purewick Corporation, El Cajon, California 92020 (US)
(72) Inventor: LAMBERT, Michael, Snellville, Georgia 30078 (US); DAVIS, Kathleen, Atlanta, Georgia 30307 (US); RAMOS RODRIGUEZ, Maria Renee, Florham Park, New Jersey 07932 (US); FRANKLIN, Jeff Edward, Hamilton, Ohio 45011 (US); DAVIS, Brandt, Milford, Ohio 45150 (US); KNOLLMAN, Kevin Michael, West Chester, Ohio 45069 (US); WESNER, Kyra Kormos, Loveland, Ohio 45140 (US); SCHNELL, Michelle, Hamilton, Ohio 45013 (US); WEIR, Mackenzie Brooke, Chardon, Ohio 44024 (US); FILBRUN, Joseph, Cincinnati, Ohio 45220 (US)
(74) Representative: Hoffmann Eitle
(86) International application number: PCT/US2021/048211
(87) International publication number: WO 2022/051220

(56) References cited:
- WO-A1-2019/212954
- US-A- 5 267 988

## Description

### CROSS-REFERENCE TO RELATED APPLICATIONS

This application claims priority to U.S. Provisional Patent Application No. 63/073,553 filed on September 2, 2020.

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, can be used to address some of these circumstances. However, bed pans and urinary catheters have several problems associated therewith. For example, bed pans can be prone to discomfort, pressure ulcers spills, and other hygiene issues. Urinary catheters be can be uncomfortable, painful, and can cause urinary tract infections.

Thus, users and manufacturers of fluid collection devices continue to seek new and improved devices, systems, and methods to collect urine. WO 2019/212954 A1 discloses a fluid collection system configured to be worn by a user and having a fluid collection device shaped to complement the female anatomy or male anatomy near the urethra.

### SUMMARY

According to a first aspect, there is provided a fluid collection system according to claim 1

[deleted]

Features from any of the disclosed embodiments may be used in combination with one another , without limitation, as long as they fall within the scope of the claims. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1A** is a front isometric view of a female wearing a fluid collection system, according to an embodiment.
**FIG. 1B** is a front isometric view of the fluid collection system of **FIG. 1A****.**
**FIG. 2A** is an isometric view of a fluid collection system, according to an embodiment.
**FIG. 2B** is a cross-sectional view of the female fluid device of **FIG. 2A** taken along line 2-2 thereof.
**FIG. 3A** is a front isometric view of a female wearing a fluid collection system, according to an embodiment.
**FIG. 3B** is a front isometric view of the fluid collection system of **FIG. 3A****.**
**FIG. 4A** is a front isometric view of a female wearing a fluid collection system, according to an embodiment.
**FIG. 4B** is a front isometric view of the fluid collection system of **FIG. 4A****.**
**FIG. 5** is a front view of a fluid collection system, according to an embodiment.
**FIG. 6** is a front view of a fluid collection system, according to an embodiment.
**FIG. 7A** is a front isometric view of a female wearing a fluid collection system, according to an embodiment.
**FIG. 7B** is a front isometric view of the fluid collection system of **FIG. 7B****.**
**FIG. 8** is a flow diagram of a method to collect fluid, according to an embodiment.
**FIG. 9** is a block diagram of a system for fluid collection, according to an embodiment.

### DETAILED DESCRIPTION

Embodiments disclosed herein are fluid collection systems. In many embodiments, a fluid collection system includes a fluid collection device and one or more straps secured or securable to the fluid collection device. The one or more straps are configured to wrap around the user to provide the technical effect of securing the fluid collection device at least proximate (*e.g*., adjacent) to the urethra of the user. The one or more straps may include straps that go around one or more of the waist, hips, buttocks, or tops of thighs of the user that result in the technical effect of holding the fluid collection device in place and prevent dislodgement of the fluid collection device. For example, the fluid collection device may include a fluid impermeable barrier and a fluid permeable body positioned at least partially within the fluid impermeable barrier to extend across at least a portion of an opening in the fluid impermeable barrier. The one or more straps provide the technical effect of retaining the fluid collection device in place with the opening of the fluid impermeable barrier and a portion of the fluid permeable body proximate or adjacent to the urethra of the user. The one or more straps may be secured or securable to the fluid impermeable barrier or the conduit of the fluid collection device. The one or more straps may be secured to a patch that covers a separate fluid collection device and holds the fluid collection device in place over the urethra of the user. In at least one, some, or all of the embodiments disclosed herein, the one or more straps result in the technical effect of effectively securing the fluid collection device in place in a variety of patients having unique physiological characteristics, including patients having a less gluteal or labial tissue.

The fluid collection systems disclosed herein are configured to collect fluids from an individual. The fluids collected by the fluid collection devices may include urine. The fluids collected by the fluid collection devices may also include at least one of vaginal discharge, penile discharge, reproductive fluids, blood, sweat, or other bodily fluids.

Fluid collection systems described herein may be used in fluid collection systems. In addition to the fluid collection device and the one or more straps, the fluid collection systems may include a fluid storage container and a portable vacuum source. Fluid (*e.g.,* urine or other bodily fluids) collected in the fluid collection device may be removed from the fluid collection device via a conduit which protrudes into an interior region of the fluid collection device. For example, a first open end of the conduit may extend into the fluid collection device to a reservoir therein. The second open end of the conduit may extend into the fluid collection device or the portable vacuum source. The suction force may be introduced into the interior region of the fluid collection device via the first open end of the conduit responsive to a suction (*e.g*., vacuum) force applied at the second end of the conduit. The suction force may be applied to the second open end of the conduit by the portable vacuum source either directly or indirectly.

Fluid collection systems described herein may be shaped and sized to be positioned adjacent to the opening of a female urethra or positioned over the urethra opening of a male having a buried penis. For example, the fluid collection device may include a fluid impermeable barrier at least partially defining a chamber (*e.g*., interior region of the fluid collection device) of the fluid collection device. The fluid impermeable barrier also defines an opening extending therethrough from the external environment. The opening may be positioned adjacent to a female urethra or over a male urethra. The fluid collection device may include a fluid permeable body disposed within the fluid impermeable barrier. The conduit may extend into the fluid collection device at a first end region, through one or more of the fluid impermeable barrier, fluid permeable body to a second end region of the fluid collection device. Exemplary fluid collection devices for use with the systems and methods herein are described in more detail below.

In some embodiments, the portable vacuum source may be disposed in or on the fluid collection device. In such embodiments, the conduit may extend from the fluid collection device and attach to the portable vacuum source at a first point therein. An additional conduit may attach to the portable vacuum source at a second point thereon and may extend out of the fluid collection device, and may attach to the fluid storage container. Accordingly, a vacuum (*e.g*., suction) may be drawn through the fluid collection device via the fluid storage container. Fluid, such as urine, may be drained from the fluid collection device using the portable vacuum source.

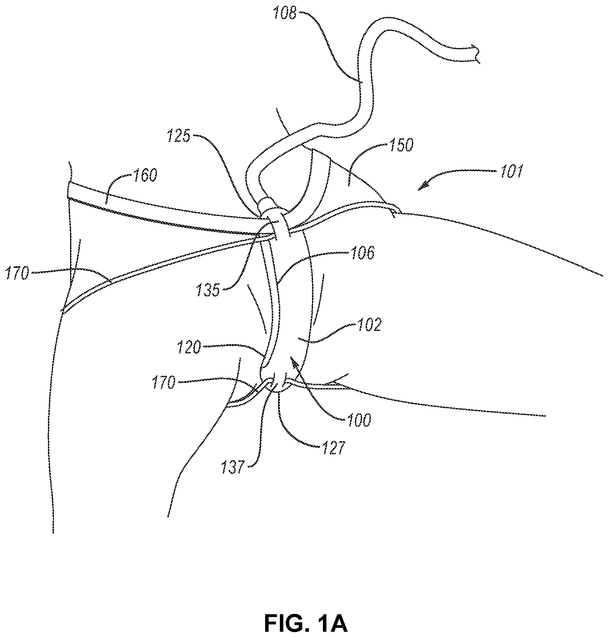

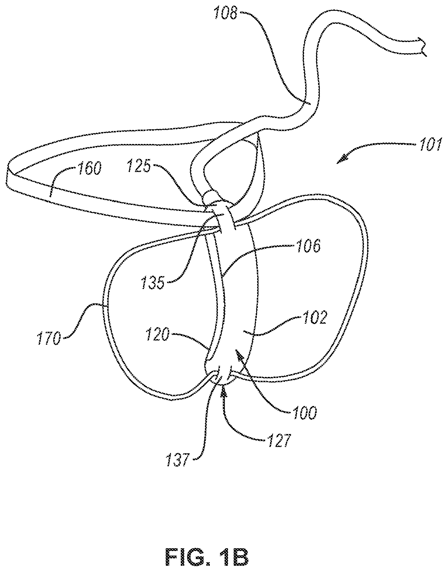

**FIG. 1A** is an isometric view of fluid collection system 101 worn by a user 150, and **FIG. 1B** is an isometric view of the fluid collection system 101 without the user 150, according to an embodiment. The fluid collection device 100 is an example of a female fluid collection device 100 that is configured to receive fluids from a female. The fluid collection system 101 includes fluid collection device 100, a waist strap 160, and one or more thigh straps 170. The fluid collection device 100 includes a fluid impermeable barrier 102 having a first end region 125 and a second end region 127 distal to the first end region 125. The fluid impermeable barrier 102 also defines an opening 106 positioned between the first end region 125 and the second end region 127. The fluid collection device 100 also includes a fluid permeable body 120 positioned within a chamber defined at least partially by the fluid impermeable barrier 102. The fluid permeable body 120 is positioned within the chamber to extend across at least a portion (e.g., all) of the opening 106 of the fluid impermeable barrier 102. The fluid collection device 100 also may include a conduit 108 extending through an aperture in the first end region 125 of the fluid impermeable barrier 102.

The fluid collection device 100 also includes one or more fasteners or securement elements configured to secure the waist strap 160 and the one or more thigh straps 170 to the fluid collection device 100. For example, the fluid impermeable barrier 102 includes a first strip 135 proximate to the first end region 125 and a second strip 137 proximate to the second end region 127. The first strip 135 and the second strip 137 each at least partially define a through hole or slit between at least a portion of the respective strip 135, 137 and the fluid impermeable barrier 102. At least one (*e.g.,* both) of the first strip 135 and the second strip 137 may be molded to or with the fluid impermeable barrier 102, and may include the same or similar material as the fluid impermeable barrier 102. In some embodiments, at least one (*e.g.,* both) of the first strip 135 and the second strip 137 may be secured or securable to the fluid impermeable barrier 102 with an adhesive or a mechanical attachment. In the fluid collection device 100, at least one (*e.g.,* both) of the first strip 135 and the second strip 137 are positioned on a side or a surface of the fluid impermeable barrier 102 that is generally opposite or distal to the opening 106. In some embodiments, at least one of the first strip 135 or the second strip 137 may be positioned elsewhere on the fluid impermeable barrier 102. For example, the first strip 135 may be positioned on the fluid impermeable barrier 102 at the first end region 125 between the aperture and the opening 106. The second strip 137 may be positioned on the fluid impermeable barrier at the second end region 127 between a distal end of the fluid impermeable barrier 102 and the opening 106.

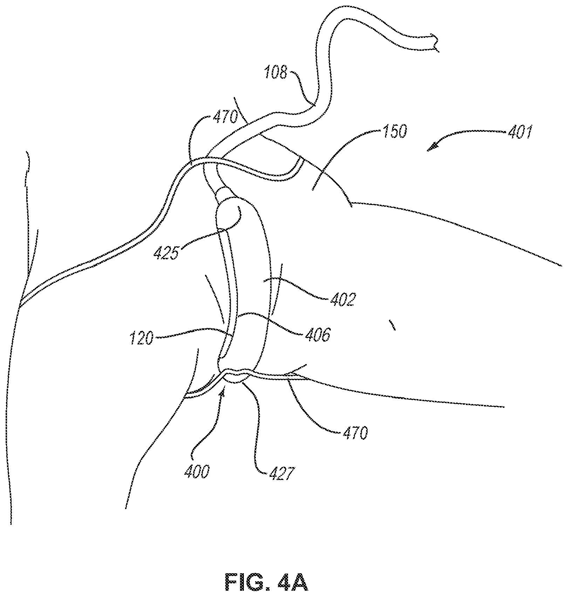

The fluid collection system 101 also includes waist strap 160 secured or securable to the fluid collection device 100 and configured to fit around a waist or hips of the user 150. In the fluid collection system 101, the waist strap 160 is secured to the first end region 125 of the fluid impermeable barrier 102 with the first strip 135. For example, the waist strap 160 may be positioned or fed through a through hole between the first strip 135 and the fluid impermeable barrier 102. In some embodiments, the waist strap 160 is releasably secured or securable to the fluid collection device 100. In some embodiments, the waist strap 160 is fixedly secured to the fluid collection device 100. While the waist strap 160 is secured or securable to the first end region 125 of the fluid impermeable barrier 102 in the fluid collection system 100, the waist strap 160 may be secured elsewhere on the fluid impermeable barrier 102 in other embodiments, such as between the first end region 125 and the second end region 127, proximate to the second end region 127, generally distal to the opening 106, and/or generally proximate to the opening 106. In some embodiments, the waist strap 160 may be secured to the conduit 108, similar to the waist strap 470 shown in **FIGS. 4A-4B****.**

In some embodiments, the waist strap 160 may be elastic or otherwise stretchable to stretch and fit around the waist or hips of the user 150. In some embodiments, the waist strap 160 may include a fastener configured to fasten two portions or ends of the waist strap 160 together to fit the waist strap 160 around the waist of the user 150. The fastener may include clips, hook and loop fasteners, buttons, snaps, a zipper, magnets, clasps ultrasonic welding, or any other fastener. In some embodiments, the size of the waist strap 160 is adjustable using one or more of the fasteners described herein. The waist strap 160 also may include both an elastic or stretchable material and one or more fasteners. In some embodiments, the waist strap 160 may include a generally flat strap or band.

The fluid collection system 101 also includes one or more thigh straps 170 secured or securable to the fluid collection device 100 and configured to fit around the one or more thighs of the user 150. In some embodiments, the one or more thigh straps 170 include two thigh straps 170 each configured to wrap around different thigh of the user 150. For example, the two thigh straps 170 and the fluid collection device 100 may form two distinct loops each configured to wrap or extend around a different thigh of the user 150 with the fluid collection device 100 positioned between the two loops. In some embodiments, the two thigh straps 170 may include a single continuous strap secured to the fluid collection device 100 in two regions to form, with the fluid collection device 100, the two distinct loops. In some embodiments, the two thigh straps 170 include two different straps each secured or securable to the fluid collection device 100 to form the two distinct loops. In some embodiments, the one or more thigh straps 170 include a single strap forming a single loop that fits around the both the thighs of the user 150.

In the fluid collection system 101, the one or more thigh straps 170 are secured to the front end region 125 of the fluid impermeable barrier 102 with the first strip 135 and also secured to the second end region 127 of the fluid impermeable barrier 102 with the second strip 137. The one or more thigh straps 170 may be positioned or fed through through holes between the strips 135, 137 and the fluid impermeable barrier 102. In some embodiments, the one or more thigh straps 170 are releasably secured or securable to the fluid collection device 100. In some embodiments, the one or more thigh straps 170 are fixedly secured to the fluid collection device 100. While the one or more thigh straps 170 are secured or securable to the first end region 125 and the second end region 127 of the fluid impermeable barrier 102 in the fluid collection system 100, the one or more thigh straps 170 may be secured or securable elsewhere on the fluid impermeable barrier 102 in other embodiments, such as between the first end region 125 and the second end region 127, generally distal to the opening 106, and/or generally proximate to the opening 106. In some embodiments, the one or more thigh straps may be secured or securable to the conduit 108 and any of the described positions on the fluid impermeable barrier 102, similar to the waist strap 470 shown in **FIGS. 4A-4B****.**

In some embodiments, the one or more thigh straps 170 may be elastic or otherwise stretchable to stretch to fit around the waist of the user 150. In some embodiments, the one or more thigh straps 170 may include a fastener configured to fasten two portions or ends of the one or more thigh straps 170 together to fit the one or more thigh straps 170 around one or more thighs of the user 150. The fastener may include clips, hook and loop fasteners, buttons, snaps, a zipper, magnets, clasps, ultrasonic welding, or any other fastener. In some embodiments, the size of the one or more thigh straps 170 is adjustable using one or more of the fasteners described herein. The one or more thigh straps 170 also may include both an elastic or stretchable material and one or more fasteners. In some embodiments, the one or more thigh straps 170 may be generally string-like. At least one (e.g., both) of the one or more thigh straps 170 and the waist strap 160 may be detachably secured or securable to the fluid collection device 100 such that the fluid collection device 100 may be used with different thigh straps 170 and/or waist straps 160, such as different sizes of thigh straps 170 and/or waist straps 160. Moreover, if at least one of the thigh straps 170 or the waist strap 160 becomes defective or soiled, the at least one of the thigh straps 170 or the waist strap 160 may be replaced with a new thigh strap 170 or waist strap 160 without disposing of the fluid collection device 100.

In use, the legs of the user 150 may be inserted through the loop defined at least partially by the waist strap 160 and the one or more loops at least partially defined by the one or more thigh straps. The fluid collection assembly 101 may then be moved to position the fluid permeable body 120 of the fluid collection device 100 proximate to the urethra of the user 150 with the waist strap 160 positioned around the waist or hips of the user 150 and the one or more thigh straps 170 positioned around the thighs of the user 150. The waist strap 160 and/or the one or more thigh straps 170 provide the technical effect of retaining the fluid collection device 100 in place with the fluid permeable body 120 positioned at least proximate (e.g., adjacent) to the urethra of the user 150 through the opening 106 in the fluid impermeable barrier 102.

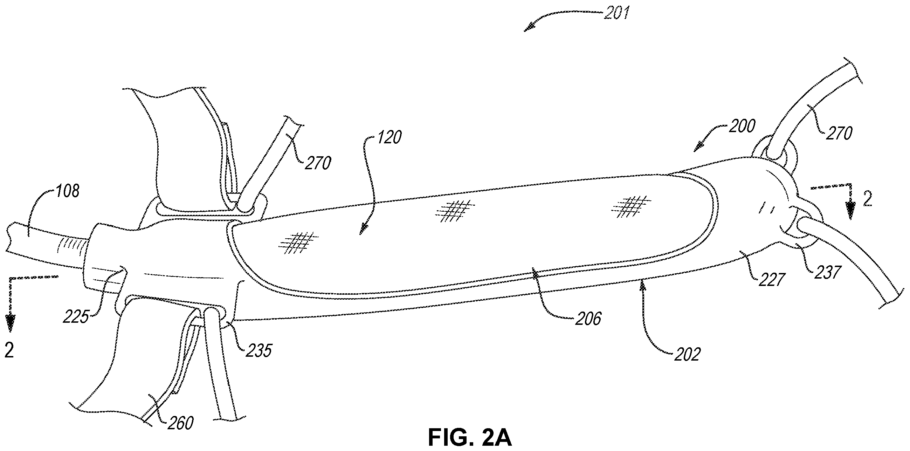

**FIG. 2A** is an isometric view of a fluid collection system 201 including a fluid collection device 200, a waist strap 260, and one or more thigh straps 270. Unless otherwise noted, the waist strap 260 and the one or more thigh straps 270 may include any aspect of the waist strap 160 and the one or more thigh straps 160 described above in relation to the fluid collection system 101. The fluid collection device 200 also may include any aspect of the fluid collection device 100, including the fluid permeable body 120 and a fluid impermeable barrier 202 defining an opening 206 and having a first end region 225 and a second end region 227.

The fluid collection device 200 also includes fasteners configured to secure the waist strap 260 and the one or more thigh straps 270 to the fluid collection device 200. For example, the fluid collection device 200 includes one or more first eyelets 235 at the first end region 225 and one or more second eyelets 237 at the second end region 227. At least one (*e.g*., both) of the eyelets 235, 237 may be molded to or as part of the fluid impermeable barrier 202. As such, at least one (*e.g*., both) of the eyelets 235, 237 may include material that is substantially the same or similar to the fluid impermeable barrier 202. In some embodiments, the eyelets 235, 237 may be secured or securable to the fluid impermeable barrier 202 with an adhesive or mechanical attachment.

In the fluid collection system 201, the fluid collection device 200 includes two first eyelets 235 positioned at the first end region 225 of the fluid impermeable barrier 202. The two first eyelets 235 are positioned generally opposite to one another at the first end region 235 and may be approximately on a plane with the elongated edges of the opening 206. In some embodiments, the two first eyelets 235 are positioned generally opposite to one another with one first eyelet positioned between the aperture and the opening 206 and another first eyelet positioned on a side of the fluid impermeable barrier 202 generally opposite to the opening 206. In some embodiments, the fluid collection device 200 includes only a single first eyelet 235 positioned or positionable at the first end region 225. The single first eyelet 235 may be positioned or positionable between the opening 206 and the aperture, or may be positioned on a side of the fluid impermeable barrier 202 generally opposite to the opening 206. The one or more first eyelets also may be elsewhere on the fluid impermeable barrier 202, such as generally opposite to the opening 206 between the first end region 225 and the second end region 227. In some embodiments, one or more first eyelets 235 may be positioned or positionable on the conduit 108.

In the fluid collection system 201, each of the one or more first eyelets 235 secures both the waist strap 260 and the one or more thigh straps 270 to the first end region 225 of the fluid impermeable barrier 202. In some embodiments, a different eyelet may secure each of the waist strap 260 and the one or more thigh straps 270 to the fluid impermeable barrier 202.

In the fluid collection system 201, the fluid collection device 200 includes two second eyelets 237 positioned at the second end region 227 of the fluid impermeable barrier 202. The two second eyelets 237 are positioned or positionable generally opposite to one another at the second end region 235 and may be approximately on a plane with the elongated edges of the opening 206. In some embodiments, the two second eyelets 237 are positioned or positionable generally opposite to one another with one second eyelet positioned between the aperture and the opening 206 and another second eyelet positioned on a side of the fluid impermeable barrier 202 generally opposite to the opening 206. With two first eyelets 235 and two second eyelets 237, the one or more thigh straps include two thigh straps 270 each secured to a different first eyelet 235 and a different second eyelet 237.

In some embodiments, the fluid collection device 200 includes only a single second eyelet positioned at the second end region 227. The single second eyelet may be positioned or positionable between the opening 206 and the distal end of the fluid impermeable barrier 202, or may be positioned or positionable on a side of the fluid impermeable barrier 202 generally opposite to the opening 206. The one or more second eyelets also may be elsewhere on the fluid impermeable barrier 202, such as generally opposite to the opening 206 between the first end region 225 and the second end region 227. In embodiments having a single second eyelet 237, the one or more thigh straps 270 may include a single thigh strap fed through or otherwise secured to the single second eyelet 237.

While eyelets are shown on the fluid collection device 200, other embodiments of the fluid collection system 201 may include other fasteners secured or securable to the fluid impermeable barrier 202 in any of the positions or configurations described above. For example, clips, hook and loop fasteners, buttons, snaps, a zipper, magnets, clasps or any other fastener may be secured or secured to the fluid impermeable barrier 202. At least one (e.g., both) of the one or more thigh straps 270 and the waist strap 260 may be detachably secured or securable to the fluid collection device 200 such that the fluid collection device 200 may be used with different thigh straps 170 and/or waist straps 160, such as different sizes of thigh straps 270 and/or waist straps 260. Moreover, if at least one of the thigh straps 270 or the waist strap 260 becomes defective or soiled, the at least one of the thigh straps 270 or the waist strap 260 may be replaced with a new thigh strap 270 or waist strap 260 without disposing of the fluid collection device 200.

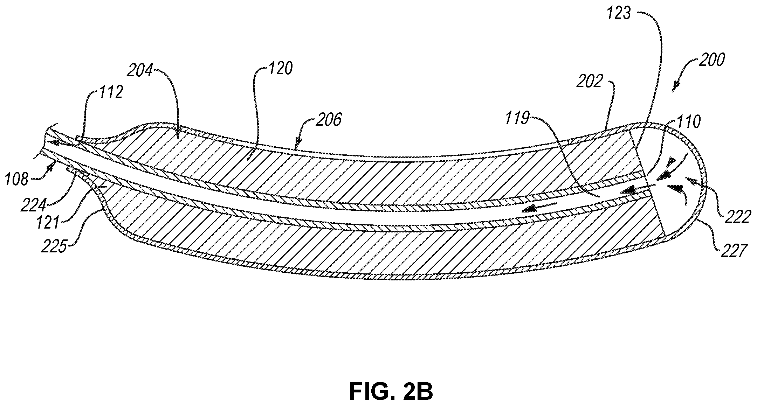

**FIG. 2B** is a cross-sectional view of the fluid collection device 200 taken along line 2-2 of **FIG. 2A****.** Unless otherwise noted, any of the fluid collection devices described herein, including the fluid collection device 100, may include any aspect of the fluid collection device 200 described herein. The fluid impermeable barrier 202 at least partially defines a chamber 204 (*e.g.,* interior region) and includes an inward border or edge defining an opening 106. The fluid impermeable barrier 202 is substantially cylindrical in shape between the first end region 225 and the second end region 227. In other embodiments, the fluid impermeable barrier 202 may include other shapes, such as one of more substantially planar surfaces, triangular, or other suitable shapes. The opening 206 is formed in and extends longitudinally through the fluid impermeable barrier 202, thereby enabling fluids to enter the chamber 204 from outside of the fluid collection device 200. The opening 206 may be configured to be positioned at least proximate (*e.g*., adjacent) to the opening of a female urethra.

The fluid collection device 200 may be positioned at least proximate to the opening of the female urethra and urine may enter the interior region of the fluid collection device 200 via the opening 206. The fluid collection device 200 is configured to receive the fluids into the chamber 204 via the opening 206. For example, the opening 206 may exhibit an elongated shape that is configured to extend from a first location below the urethral opening (*e.g.,* at or near the anus or the vaginal opening) to a second location above the urethral opening (*e.g.,* at or near the clitoris or the pubic hair). The opening 206 may exhibit an elongated shape since the space between the legs of a female is relatively small when the legs of the female are closed, thereby only permitting the flow of the fluids along a path that corresponds to the elongated shape of the opening 206. For example, the opening may extend longitudinally along the fluid impermeable barrier. The opening 206 in the fluid impermeable barrier 202 may exhibit a width that is measured transverse to the longitudinal direction and may be at least about 10% of the circumference of the fluid collection device 100, such as about 25% to about 50%, about 40% to about 60%, about 50% to about 75%, about 65% to about 85%, or about 75% to about 100% of the circumference of the fluid collection device 200. The opening 206 may exhibit a width that is greater than 50% of the circumference of the fluid collection device 200 since the vacuum (*e.g.,* suction) through the conduit 108 pulls the fluid into the conduit 108. In some embodiments, the opening 206 may be vertically oriented (*e.g.,* having a major axis parallel to the longitudinal axis of the device 200). In some embodiments, (not shown), the opening 206 may be horizontally oriented (*e.g.,* having a major axis perpendicular to the longitudinal axis of the device 200). In some embodiments, the inward border or edge 129 of the fluid impermeable barrier 202 defines the opening 206. The edge may include two opposing arced portions, the arc portions following the outer circumference or periphery of the substantially cylindrical fluid impermeable barrier 202. In an embodiment, the fluid impermeable barrier 202 may be configured to be attached to the individual, such as adhesively attached (*e.g.,* with a hydrogel adhesive) to the individual. According to an embodiment, a suitable adhesive is a hydrogel layer, such as those disclosed in U.S. Patent Application Publication No. 2017/0189225.

The fluid impermeable barrier 202 may also temporarily store the fluids in the chamber 204. For example, the fluid impermeable barrier 202 may be formed of any suitable fluid impermeable materials, such as a fluid impermeable polymer (*e.g.,* silicone, polypropylene, polyethylene, polyethylene terephthalate, a polycarbonate, etc.), polyurethane films, thermoplastic elastomer (TPE), rubber, thermoplastic polyurethane, another suitable material, or combinations thereof. As such, the fluid impermeable barrier 202 substantially prevents the fluids from exiting the portions of the chamber 204 that are spaced from the opening 206. The fluid impermeable barrier 202 is flexible, thereby enabling the fluid collection device 200 to bend or curve when positioned against the body of a wearer. Example fluid impermeable barriers may include, but are not limited to, a fluid impermeable barrier including at least one of Versa flex CL 2000X TPE, Dynaflex G6713 TPE, or Silpuran 6000/05 A/B silicone.

In an embodiment, the fluid impermeable barrier 202 may be air permeable. In such an embodiment, the fluid impermeable barrier 202 may be formed of a hydrophobic material that defines a plurality of pores. In an embodiment, one or more portions of at least the outer surface of the fluid impermeable barrier 202 may be formed from a soft and/or smooth material, thereby reducing chaffing. The fluid impermeable barrier 202 may include markings thereon, such as one or more markings to aid a user in aligning the device 200 on the wearer. For example, a line on the fluid impermeable barrier 202 (*e.g.,* opposite the opening 206) may allow a healthcare professional to align the opening 206 over the urethra of the wearer. In examples, the markings may include one or more of alignment guide or an orientation indicator, such as a stripe or hashes. Such markings may be positioned to align the device 200 to one or more anatomical features such as a pubic bone, etc.

The fluid collection device 200 may include the fluid permeable body 120 or layer disposed in the chamber 204. Any fluid collection assembly and fluid collection device described herein may utilize any aspect of the fluid permeable body 120. The fluid permeable body 120 may cover or extend across at least a portion (*e.g.,* all) of the opening 206. The fluid permeable body 120 may be configured to wick any fluid away from the opening 106, thereby preventing the fluid from escaping the chamber 204. The fluid permeable body 120 also may wick the fluid generally towards an interior of the chamber 204, as discussed in more detail below. A portion of the fluid permeable body 120 may define a portion of an outer surface of the fluid collection device 200. Specifically, the portion of the fluid permeable body 120 defining the portion of the outer surface of the fluid collection device 200 may be the portion of the fluid permeable body 120 exposed by the opening 206 defined by the fluid impermeable barrier 202 that contacts the user. Moreover, the portion of the fluid permeable device defining the portion of the outer surface of the fluid collection device 200 may be free from coverage by gauze or other wicking material at the opening.

The fluid permeable body 120 can be configured to wick and/or allow transport of fluid away from the opening 206 towards a reservoir 222 and/or an inlet 110 of the conduit 108. The fluid permeable body 120 may include any material that may wick the fluid. The permeable properties referred to herein may be wicking, capillary action, diffusion, or other similar properties or processes, and are referred to herein as "permeable" and/or "wicking." Such "wicking" or other physical properties may exclude absorption into the fluid permeable body 120, such as not include absorption of the bodily fluid into the fluid permeable body 120. Put another way, substantially no absorption or solubility of the bodily fluids into the material may take place after the material is exposed to the bodily fluids and removed from the bodily fluids for a time. While no absorption or solubility is desired, the term "substantially no absorption" may allow for nominal amounts of absorption and/or solubility of the bodily fluids into the fluid permeable body 120 (e.g., absorbency), such as less than about 30 wt% of the dry weight of the fluid permeable body 110, less than about 20 wt%, less than about 10 wt%, less than about 7 wt%, less than about 5 wt%, less than about 3 wt%, less than about 2 wt%, less than about 1 wt%, or less than about 0.5 wt% of the dry weight of the fluid permeable body 210. In an embodiment, the fluid permeable body 210 may include at least one absorbent or adsorbent material.

The fluid permeable body 120 may include a one-way fluid movement fabric. As such, the fluid permeable body 120 may remove fluid from the area around the female urethra, thereby leaving the urethra dry. The fluid permeable body 120 may enable the fluid to flow generally towards the reservoir 222 of void space formed within the chamber 204 and/or the inlet 110 of the conduit 108. For example, the fluid permeable body 120 may include a porous or fibrous material, such as hydrophilic polyolefin. In some embodiments, the fluid permeable body 120 consists of or consists essentially of a porous or fibrous material, such as hydrophilic polyolefin. Examples of polyolefin that may be used in the fluid permeable body 120 include, but are not limited to, polyethylene, polypropylene, polyisobutylene, ethylene propylene rubber, ethylene propylene diene monomer, or combinations thereof. The porous or fibrous material may be extruded into a substantially cylindrically shape to fit within the chamber 204 of the fluid impermeable barrier 102. The fluid permeable body 120 may include varying densities or dimensions. Moreover, the fluid permeable body 120 may be manufactured according to various manufacturing methods, such as molding, extrusion, or sintering.

In some embodiments, the fluid permeable body 120 includes a singular and porous body. That is, during use, the fluid permeable body 120 extends from the conduit 108 to interface the fluid impermeable barrier 202 and the opening 206. In some embodiments, a majority of the outer surface of the fluid permeable body 120 interfaces with an inner surface of the fluid impermeable barrier 202. A singular fluid permeable body 120 may be advantageous to conventional systems, which typically require an air-laid nonwoven pad covered by a ribbed fabric compression bandage, because a singular fluid permeable body 120 reduced the number of components in the fluid collection device 200, reduces the assembly time of the fluid collection device 200, requires shelf-life data for only a single component, and provides a latex-free single component. In some embodiments, at least a portion of the singular porous material of the fluid permeable body 120 extends continuously between the opening 206 and the reservoir 222 to wick any fluid from the opening 206 directly to the reservoir 222. Moreover, as the fluid impermeable barrier 202 is flexible and the fluid permeable body 120 is configured to wick fluid from the body rather than absorb fluid from the body and hold the fluid against the body, the fluid collection device 200, in some embodiments, is free from a seal or cushioning ring on the inward edge defining the opening 206. In these and other embodiments, the fluid permeable body 120 includes an outer surface and a single layer or type of material between the opening 206 and the conduit 108 positioned within the fluid permeable body 120.

In some embodiments, the fluid permeable body 120 may include two or more layers of fluid permeable materials and may include no (or an absence of) more than two layers of material between the opening 206 and the conduit 108 positioned within the fluid permeable body 120. For example, the fluid collection device 100 may include a fluid permeable membrane covering or wrapping around at least a portion of a fluid permeable body, with both the fluid permeable membrane and the fluid permeable body being disposed in the chamber 204. The fluid permeable membrane may cover or extend across at least a portion (*e.g.,* all) of the opening 206. The fluid permeable membrane may be configured to wick any fluid away from the opening 206, thereby preventing the fluid from escaping the chamber 204. The permeable properties referred to herein may be wicking, capillary action, diffusion, or other similar properties or processes, and are referred to herein as "permeable" and/or "wicking." In some embodiments, at least one of the fluid permeable membrane or the fluid permeable support include nylon configured to wick fluid away from the opening 206. The material of the fluid permeable membrane and the fluid permeable support also may include natural fibers. In such examples, the material may have a coating to prevent or limit absorption of fluid into the material, such as a water repellent coating.

The fluid permeable membrane may also wick the fluid generally towards an interior of the chamber 204. The fluid permeable membrane may include any material that may wick the fluid. For example, the fluid permeable membrane may include fabric, such as a gauze (*e.g.,* a silk, linen, polymer based materials such as polyester, or cotton gauze), another soft fabric (*e.g.,* jersey knit fabric or the like), or another smooth fabric (*e.g.,* rayon, satin, or the like). Forming the fluid permeable membrane from gauze, soft fabric, and/or smooth fabric may reduce chaffing caused by the fluid collection device 200. Other embodiments of fluid permeable membranes, fluid permeable supports, chambers, and their shapes and configurations are disclosed in U.S. Patent Application No. 15/612,325 filed on June 2, 2017; U.S. Patent Application No. 15/260,103 filed on September 8, 2016; U.S. Patent Application No. 15/611,587 filed on June 1, 2017; PCT Patent Application No. PCT/US19/29608, filed on April 29, 2019. In many embodiments, the fluid permeable body 120 includes a fluid permeable support including a porous spun nylon fiber structure and a fluid permeable wicking membrane including gauze at least partially enclosing the spun nylon fiber structure. For example, the fluid permeable body 120 may include a gauze or other wicking fabric positioned to contact the skin of the user through the opening 106. In some embodiments, the gauze or other wicking fabric is wrapped around a body of spun nylon fibers material and/or covering both sides of a substantially planar spun nylon fibers material. In some embodiments, the gauze or other wicking fabric covers the side of substantially planar spun nylon fibers material that is oriented towards the skin of the user.

The fluid collection device 200 also includes conduit 108 that is at least partially disposed in the chamber 204. The conduit 108 (*e.g.,* a tube) includes an inlet 110 at a second end region 227 of the fluid impermeable barrier 202 and an outlet 112 at or outside the first end region 225 of the fluid impermeable barrier 202 positioned downstream from the inlet 110. The conduit 108 provides fluid communication between an interior region of the chamber 204 and a fluid storage container (not shown) or a portable vacuum source (not shown). For example, the conduit 108 may directly or indirectly fluidly couple the interior region of the chamber 204 and/or the reservoir 222 with the fluid storage container or the portable vacuum source.

In the illustrated embodiment, the fluid permeable body 120 defines a bore 119 extending through the fluid permeable body 120 from a first body end 121 of the fluid permeable body 120 to a second body end 123 of the fluid permeable body 120 distal to the first body end 120. In other embodiments, the bore 119 extends only partially into the fluid permeable body from the first body end 121 of the fluid permeable body 120.

In the illustrated embodiment, the conduit 108 is at least partially disposed in the chamber 204 and interfaces at least a portion of the bore 119 of the fluid permeable body 120. For example, the conduit 108 may extend into the fluid impermeable barrier 202 from the first end region 225 (*e.g.,* proximate to the outlet 112) and may extend through the bore 119 to the second end region 227 (*e.g.,* opposite the first end region 225) to a point proximate to the reservoir 222 such that the inlet 110 is in fluid communication with the reservoir 222. For example, in the illustrated embodiment, the inlet 110 is positioned flush with the end 123 of the fluid permeable body 120 that partially defines the reservoir 222. However, in other embodiments, the inlet 110 is positioned in the reservoir 222 or recessed from the end 123 of the fluid permeable body 120. The fluid collected in the fluid collection device 100 may be removed from the interior region of the chamber 204 via the conduit 108. The conduit 108 may include a flexible material such as plastic tubing (*e.g.,* medical tubing). Such plastic tubing may include a thermoplastic elastomer, polyvinyl chloride, ethylene vinyl acetate, polytetrafluoroethylene, etc., tubing. In some embodiments, the conduit 108 may include silicone or latex.

The fluid impermeable barrier 202 may store fluids in the reservoir 222 therein. The reservoir 222 may be an unoccupied portion of the chamber 204 and is void of other material. In some embodiments, the reservoir 222 is defined at least partially by the fluid permeable body 120 and the fluid impermeable barrier 202. For example, in an embodiment, the reservoir 222 may be located at the portion of the chamber 204 that is closest to the inlet 110 (*e.g.,* the second end region). Accordingly, in the embodiment in **FIG. 2A****,** the reservoir 222 is defined by the second body end 123 of the fluid permeable body 120 and the second end region 227 of the fluid impermeable barrier 202. However, the reservoir 222 may be located at different locations in the chamber 204. For example, the reservoir 222 may be located at the end of the chamber 204 that is closest to the outlet 112. In these and other embodiments, the conduit 108 may extend through the first end region 225 of the fluid impermeable barrier 202 and to the reservoir 222 without extending through the fluid permeable body 120. Accordingly, in these and other embodiments, the fluid permeable body 120 may be free from the bore. In another embodiment, the fluid collection device 200 may include multiple reservoirs, such as a first reservoir that is located at the portion of the chamber of the chamber 204 that is closest to the inlet 110 (*e.g.,* second end region) and a second reservoir that is located at the portion of the of the chamber 204 that is closest to the outlet 112 (*e.g.,* first end region). In another example, the fluid permeable body 120 is spaced from at least a portion of the conduit 108 and the reservoir 222 may be the space between the fluid permeable body 120 and the conduit 108. In some embodiments, the fluid permeable body 120 fills or occupies substantially all of the chamber 204, including filling or occupying substantially all of the reservoir 222 is between the inlet 110 and the second end region 227 of the fluid impermeable barrier 202. Other embodiments of reservoirs, fluid impermeable barriers, fluid permeable membranes, fluid permeable bodies, chambers, and their shapes and configurations are disclosed in U.S. Patent Application No. 15/612,325 filed on June 2, 2017; U.S. Patent Application No. 15/260,103 filed on September 8, 2016; and U.S. Patent Application No. 15/611,587 filed on June 1, 2017.

The fluid impermeable barrier 202 and the fluid permeable body 120 may be configured to have the conduit 108 at least partially disposed in the chamber 204. For example, the fluid permeable body 120 may be configured to form a space that accommodates the conduit 108, such as the bore 119. In another example, the fluid impermeable barrier 202 may define an aperture 224 sized to receive the conduit 108 (*e.g.,* at least one tube). The at least one conduit 108 may be disposed in the chamber 204 via the aperture 224. The aperture 224 may be configured to form an at least substantially fluid tight seal against the conduit 108 or the at least one tube thereby substantially preventing the fluids from escaping the chamber 204.

In some embodiments, the conduit 108 may extend through the fluid permeable body 120 and at least partially into the reservoir 222. In some embodiments, the conduit 108 may extend through the fluid permeable body 120 and terminate at or before the second body end 123 of the fluid permeable body 120 such that the conduit 108 does not extend into the reservoir 222 (or the reservoir 222 is absent of the conduit 108). For example, as shown in FIG. 2B, an end (*e.g*., the inlet 110) of the conduit 108 may be generally flush or coplanar with the second body end 123 of the fluid permeable body 120. In other embodiments, the end (*e.g.,* the inlet 110) of the conduit 108 may be recessed from the second body end 123 of the fluid permeable body 120. The end of the conduit 108 also may be selectively moveable between partially extending into the reservoir 222 and recessed from or flush with the second body end 123 of the fluid permeable body 120.

When secured to the fluid collection device 200, the conduit 108 is configured to provide fluid communication with and at least partially extend between one or more of a fluid storage containers (not shown) and a portable vacuum source (not shown). For example, the conduit 108 may be configured to be fluidly coupled to and at least partially extend between one or more of the fluid storage containers and the portable vacuum source. In an embodiment, the conduit 108 is configured to be directly connected to the portable vacuum source (not shown). In such an example, the conduit 108 may extend from the fluid impermeable barrier 202 by at least one foot, at least two feet, at least three feet, or at least six feet. In another example, the conduit 108 is configured to be indirectly connected to at least one of the fluid storage container (not shown) or the portable vacuum source (not shown). In some examples, the conduit may be frosted or opaque (*e.g.,* black) to obscure visibility of the fluids therein. In some embodiments, the conduit is secured to a wearer's skin with a catheter securement device, such as a STATLOCK^{®} catheter securement device available from C. R. Bard, Inc., including but not limited to those disclosed in U.S. Patent Nos. 6,117,163; 6,123,398; and 8,211,063.

The inlet 110 and the outlet 112 are configured to provide fluid communication (*e.g.,* directly or indirectly) between the portable vacuum source (not shown) and the chamber 204 (*e.g.,* the reservoir 222). For example, the inlet 110 and the outlet 112 of the conduit 108 may be configured to directly or indirectly fluidly couple the portable vacuum source to the reservoir 222. In an embodiment, the inlet 110 and/or the outlet 112 may form a male connector. In another example, the inlet 110 and/or the outlet 112 may form a female connector. In an embodiment, the inlet 110 and/or the outlet 112 may include ribs that are configured to facilitate secure couplings. In an embodiment, the inlet 110 and/or the outlet 112 may form a tapered shape. In an embodiment, the inlet 110 and/or the outlet 112 may include a rigid or flexible material.

Locating the inlet 110 at or near a gravimetrically low point of the chamber 204 enables the conduit to receive more of the fluids than if inlet 110 was located elsewhere and reduce the likelihood of pooling (*e.g.,* pooling of the fluids may cause microbe growth and foul odors). For instance, the fluids in the fluid permeable body 120 may flow in any direction due to capillary forces. However, the fluids may exhibit a preference to flow in the direction of gravity, especially when at least a portion of the fluid permeable body 120 is saturated with the fluids.

As the portable vacuum source applies a vacuum/suction in the conduit 108, the fluid(s) in the chamber 204 (*e.g.,* such as in the reservoir 222 positioned at the first end region 225, the second end region 227, or other intermediary positions within the chamber 204) may be drawn into the inlet 110 and out of the fluid collection device 200 via the conduit 108.

In an embodiment, the conduit 108 is configured to be at least insertable into the chamber 204. In such an embodiment, the conduit 108 may include one or more markers on an exterior thereof that are configured to facilitate insertion of the conduit 108 into the chamber 204. For example, the conduit 108 may include one or more markings thereon that are configured to prevent over or under insertion of the conduit 108, such as when the conduit 108 defines an inlet 110 that is configured to be disposed in or adjacent to the reservoir 222. In another embodiment, the conduit 108 may include one or more markings thereon that are configured to facilitate correct rotation of the conduit 108 relative to the chamber 204. In an embodiment, the one or more markings may include a line, a dot, a sticker, or any other suitable marking. In examples, the conduit 108 may extend into the fluid impermeable barrier 202 from the first end region 225 (*e.g.,* proximate to the outlet 112) and may extend to the second end region 227 (*e.g.,* opposite the first end region) to a point proximate to the reservoir 222 such that the inlet 110 is in fluid communication with the reservoir 222. In some embodiments (not shown), the conduit 108 may enter the second end region 227 and the inlet 110 may be disposed in the second end region 227 (*e.g.,* in the reservoir 222). The fluid collected in the fluid collection device 100 may be removed from the interior region of the chamber 204 via the conduit 108. The conduit 108 may include a flexible material such as plastic tubing (*e.g.,* medical tubing) as disclosed herein. In some examples, the conduit 108 may include one or more portions that are resilient, such as having one or more of a diameter or wall thickness that allows the conduit to be flexible.

In an embodiment, one or more components of the fluid collection device 200 may include an antimicrobial material, such as an antibacterial material where the fluid collection device may contact the wearer or the bodily fluid of the wearer. The antimicrobial material may include an antimicrobial coating, such as a nitrofurazone or silver coating. The antimicrobial material may inhibit microbial growth, such as microbial growth due to pooling or stagnation of the fluids. In an embodiment, one or more components of the fluid collection device 200 (*e.g.,* impermeable barrier 202, conduit 108, etc.) may include an odor blocking or absorbing material such as a cyclodextrine containing material or a thermoplastic elastomer (TPE) polymer.

In any of the embodiments disclosed herein, the conduits 108 may include or be operably coupled to a flow meter (not shown) to measure the flow of fluids therein, one or more securement devices (*e.g.,* a StatLock securement device, not shown) or fittings to secure the conduit 108 to one or more components of the systems or devices disclosed herein (*e.g.,* portable vacuum source or fluid storage container), or one or more valves to control the flow of fluids in the systems and devices herein. In an embodiment, at least one of portion of the conduit 108 of the fluid collection devices or systems herein may be formed of an at least partially opaque material which may obscure the fluids that are present therein. For example, a first section of the conduit 108 disclosed herein may be formed of an opaque material or translucent material while a second section of the conduit 108 may be formed of a transparent material or translucent material. In some embodiments, the first section may include transparent or translucent material. Unlike the opaque or nearly opaque material, the translucent material allows a user of the devices and systems herein to visually identify fluids or issues that are inhibiting the flow of fluids within the conduit 108.

In any of the examples, systems or devices disclosed herein, the system of fluid collection device may include moisture sensors (not shown) disposed inside of the chamber of the fluid collection device. In such examples, the moisture sensor may be operably coupled to a controller or directly to the portable vacuum source, and may provide electrical signals indicating that moisture is or is not detected in one or more portions of the chamber. The moisture sensor(s) may provide an indication that moisture is present, and responsive thereto, the controller or portable vacuum device may direct the initiation of suction to the chamber to remove the fluid therefrom. Suitable moisture sensors may include capacitance sensors, volumetric sensors, potential sensors, resistance sensors, frequency domain reflectometry sensors, time domain reflectometry sensors, or any other suitable moisture sensor. In practice, the moisture sensors may detect moisture in the chamber and may provide a signal to the controller or portable vacuum source to activate the portable suction device.

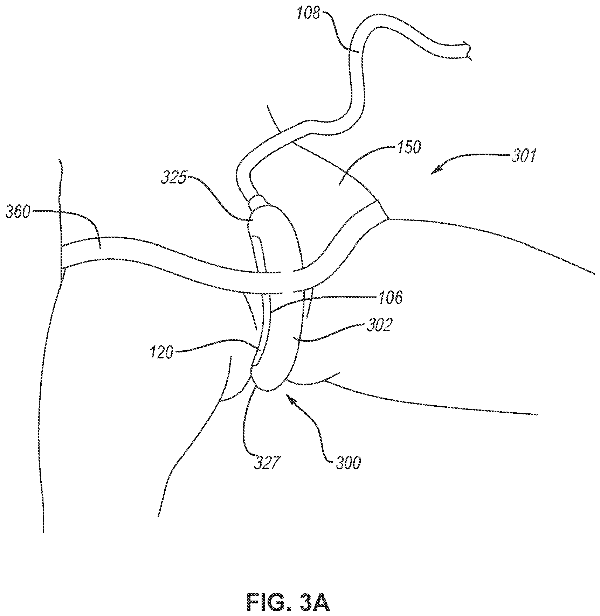

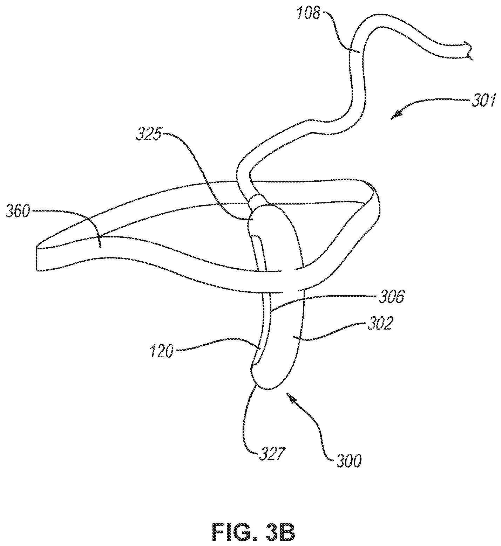

**FIG. 3A** is an isometric view of a fluid collection system 301 worn by the user 150, and **FIG. 3B** is an isometric view of the fluid collection system 301 without the user 150, according to an embodiment. The fluid collection system 301 includes a fluid collection device 300 and a waist strap 360. Unless otherwise specified, the fluid collection device 300 may include any aspect of the fluid collection devices 100, 200, described above. For example, the fluid collection device 300 includes a fluid impermeable barrier 302 having a first end region 325 and a second end region 327 distal to the first end region 325, with the fluid impermeable barrier 302 defining an opening 306 between the first end region 325 and the second end region 327. The fluid impermeable barrier 302 may include any aspect of the fluid impermeable barriers 102, 202, described above. The fluid collection device 300 also includes the fluid permeable body 120 positioned within a chamber defined by the fluid impermeable barrier 302.

The fluid collection system 301 includes the waist strap 360 secured to the fluid impermeable barrier 302, and one or more thigh straps are absent from the fluid collection system 301. The waist strap 360 may include any aspect of the waist straps 160, 260 described above. A portion of the waist strap 360 of the fluid collection system 301 is molded to and/or with the fluid impermeable barrier 302. Accordingly, at least a portion of the waist strap 360 may include material that is the same or similar to the fluid impermeable barrier 302. In some embodiments, the waist strap 360 may include an elastic or other strap secured or securable to the portion of the waist strap 360 molded to and/or with the fluid impermeable barrier 302. Although the one or more thigh straps are absent from the fluid collection system 301, in some embodiments, the one or more thigh straps may be similarly molded to or with the fluid impermeable barrier 302.

The waist strap 360 of the fluid collection system 301 is secured or securable to the fluid impermeable barrier 302 between the first end region 325 and the second end region 327. For example, the waist strap 360 may be secured or securable to the fluid impermeable barrier 302 approximately one-third of a distance between a first end at the first end region 325 and a second end at the second end region 327 of the fluid impermeable barrier 302, approximately one-half of the distance between the first end and the second end of the fluid impermeable barrier 302, or approximately two-thirds of the distance between the first end and the second end of the fluid impermeable barrier 302. The waist strap 360 may be secured or securable to the fluid impermeable barrier 302 between the opening 306 and the aperture at the first end region 325, or between the opening 306 and the second end at the second end region 327. In some embodiments, the waist strap 360 may be secured or securable to the conduit 108. The waist strap 360 may be detachably secured or securable to the fluid collection device 300 such that the fluid collection device 300 may be used with different waist straps 360, such as different sizes of waist straps 360. Moreover, if the waist strap 360 becomes defective or soiled, the at waist strap 360 may be replaced with a new thigh strap 370 or waist strap 360 without disposing of the fluid collection device 300.

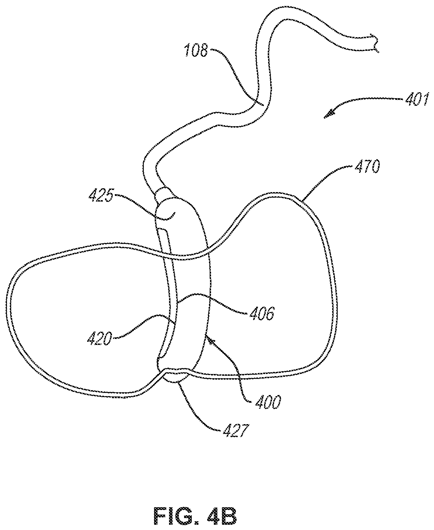

**FIG. 4A** is an isometric view of a fluid collection system 401 worn by the user 150, and **FIG. 4B** is an isometric view of the fluid collection system 401 without the user 150, according to an embodiment. The fluid collection system 401 includes a fluid collection device 400 and one or more thigh straps 470. Unless otherwise specified, the fluid collection device 400 may include any aspect of the fluid collection devices 100, 200, 300 described above. For example, the fluid collection device 400 includes a fluid impermeable barrier 402 having a first end region 425 and a second end region 427 distal to the first end region 425, with the fluid impermeable barrier 402 defining an opening 406 between the first end region 425 and the second end region 427. The fluid impermeable barrier 402 may include any aspect of the fluid impermeable barriers 102, 202, 302 described above. The fluid collection device 400 also includes the fluid permeable body 120 positioned within a chamber defined by the fluid impermeable barrier 402.

The fluid collection system 401 also includes the one or more thigh straps 470, and the waist strap is absent from the fluid collection system. The one or more thigh straps 470 may include any aspect of the one or more thigh straps 170, 270 described above. A portion of the one or more thigh straps 470 is secured or securable to fluid impermeable barrier 402 with an adhesive. The adhesive may include any adhesive known to secure the one or more thigh straps 470 to the fluid impermeable barrier 402. A portion of the one or more thigh straps 470 is secured or securable to the conduit 108 with an adhesive. In other embodiments, the portion of the one or more thigh straps 470 may be secured to the conduit 108 through molding or any of the mechanical attachments described herein or otherwise known in the art. Although the waist strap is absent from the fluid collection system 401, in some embodiments, a waist strap may be similarly secured to the fluid impermeable barrier 402 or the conduit with an adhesive. In some embodiments, the waist strap 470 may be secured to the conduit 108 through molding or any of the mechanical attachments described herein or otherwise known in the art.

The one or more thigh straps 470 of the fluid collection system 401 are secured or securable to the fluid impermeable barrier 402 at the second end region 427 and the conduit 108. Though shown in **FIGS. 4A-4B** secured to the conduit 108, in some embodiments, the one or more thigh straps 470 may be secured or securable to the first end region 425 and the second end region 427, to the fluid impermeable barrier 402 at the second end region 427 and between the first end region 425 and the second end region 427, or between the first end region 425 and the second end region 427. The one or more thigh straps 470 may be secured or securable to the fluid impermeable barrier 402 between the opening 406 and the aperture at the first end region 425 and/or between the opening 406 and the distal end at the second end region 427. In some embodiments, the one or more thigh straps 470 may be secured or securable to the conduit 108 and any of the positions on the fluid impermeable barrier 402 described above. At least one (*e.g.,* both) of the one or more thigh straps 470 and the waist strap 460 may be detachably secured or securable to the fluid collection device 400 such that the fluid collection device 400 may be used with different thigh straps 470 and/or waist straps 460, such as different sizes of thigh straps 470 and/or waist straps 460. Moreover, if at least one of the thigh straps 470 or the waist strap 460 becomes defective or soiled, the at least one of the thigh straps 470 or the waist strap 460 may be replaced with a new thigh strap 470 or waist strap 460 without disposing of the fluid collection device 400.

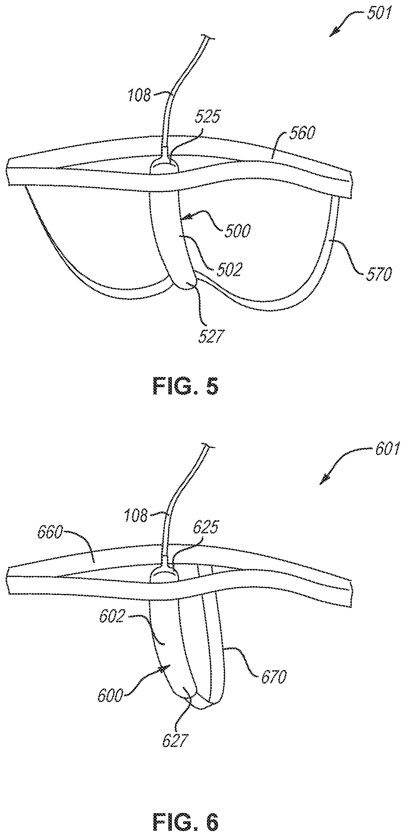

**FIG. 5** is a front view of a fluid collection system 501, according to an embodiment. The fluid collection system 501 includes a fluid collection device 500, a waist strap 560, and one or more buttocks straps 570. Unless otherwise specified, the fluid collection device 500 may include any aspect of the fluid collection devices 100, 200, 300, 400 described above. For example, the fluid collection device 500 includes a fluid impermeable barrier 502 having a first end region 525 and a second end region 527 distal to the first end region 525, with the fluid impermeable barrier 502 defining an opening (not visible) between the first end region 525 and the second end region 527. The fluid impermeable barrier 502 may include any aspect of the fluid impermeable barriers 102, 202, 302, 402 described above. The fluid collection device 500 also includes the fluid permeable body 120 (not visible) positioned within a chamber defined by the fluid impermeable barrier 502. The waist strap 560 may include any aspect of the waist straps 160, 260, 360 and may be secured or securable to the fluid impermeable barrier 502 or the conduit 108 as described above in relation to the waist straps 160, 260, 360.

The fluid collection system 501 also includes the one or more buttocks straps 570 configured to position around or on at least a portion of the buttocks of the user 150 (not shown). In some embodiments, the one or more buttocks straps 570 may be elastic or otherwise stretchable to stretch to fit around the buttocks of the user 150. In some embodiments, the one or more buttocks straps 570 include two separate buttocks straps 570 each configured to wrap or position at least partially around a different buttock of the user 150. For example, the two buttocks straps 570 may each extend between the waist strap 560 to the fluid collection device 500, with the two buttocks straps 570 angling towards one another as the two buttocks straps 570 near the fluid collection device 500. In some embodiments, the buttocks straps 570 may include a single continuous strap secured to the fluid collection device 500 between securements to the waist strap 560 in two regions.

The one or more buttocks straps 570 are secured or securable to the second end region 527 of the fluid impermeable barrier and the waist strap 560. The one or more buttocks straps 570 may be secured to the second end region 527 according to any aspect of securement of the waist straps 160, 260, 360 and/or the one or more thigh straps 170, 270, or 470 to the fluid impermeable barriers 102, 202, 302, 402 described above. For example, the one or more buttocks straps 570 may be fixedly or releasably secured to the fluid impermeable barrier 502 with one or more of an adhesive, molding, a mechanical attachment, a strip, an eyelet, ultrasonic welding, or any combination thereof. The one or more buttocks straps 570 may be secured or securable to the waist strap 560 with a threaded seam, ultrasonic welding, clips, hook and loop fasteners, buttons, snaps, a zipper, magnets, clasps or any other fastener. At least one (*e.g.,* both) of the one or more buttock straps 570 and the waist strap 560 may be detachably secured or securable to the fluid collection device 500 such that the fluid collection device 500 may be used with different buttock straps 570 and/or waist straps 560, such as different sizes of buttock straps 570 and/or waist straps 560. Moreover, if at least one of the buttock straps 570 or the waist strap 560 becomes defective or soiled, the at least one of the buttock straps 570 or the waist strap 560 may be replaced with a new buttock straps 570 or waist strap 560 without disposing of the fluid collection device 500.

In use, the legs of the user 150 may be inserted through the loop defined at least partially by the waist strap 560 and the one or more loops at least partially defined by the one or more buttocks straps 570. The fluid collection assembly 501 may then be moved to position the fluid permeable body 120 of the fluid collection device 500 proximate to the urethra of the user 150 with the waist strap 560 positioned around the waist of the user 150 and the one or more buttocks straps 570 positioned at least partially around the buttocks of the user 150. The waist strap 560 and/or the one or more buttocks straps 570 provide the technical effect of retaining the fluid collection device 500 in place with the fluid permeable body 120 positioned proximate to the urethra of the user 150 through the opening in the fluid impermeable barrier 502.

**FIG. 6** is a front view of a fluid collection system 601, according to an embodiment. The fluid collection system 601 includes a fluid collection device 600, a waist strap 660, and a buttock strap 670. Unless otherwise specified, the fluid collection device 600 may include any aspect of the fluid collection devices 100, 200, 300, 400, 500 described above. For example, the fluid collection device 600 includes a fluid impermeable barrier 602 having a first end region 625 and a second end region 627 distal to the first end region 625, with the fluid impermeable barrier 602 defining an opening (not visible) between the first end region 625 and the second end region 627. The fluid impermeable barrier 602 may include any aspect of the fluid impermeable barriers 102, 202, 302, 402, 502 described above. The fluid collection device 600 also includes the fluid permeable body 120 (not visible) positioned within a chamber defined by the fluid impermeable barrier 602. The waist strap 660 may include any aspect of the waist straps 160, 260, 360, 560 and may be secured or securable to the fluid impermeable barrier 602 or the conduit 108 as described above in relation to the waist straps 160, 260, 360, 560.

The fluid collection system 601 also includes the buttock strap 670 configured to position between the buttocks of the user 150 (not shown), such as in the gluteal cleft of the user 150. In some embodiments, the buttock strap 670 may be elastic or otherwise stretchable to stretch to fit between the buttocks of the user 150. The buttock strap 670 is secured or securable to the second end region 627 of the fluid impermeable barrier and the waist strap 660. The buttock strap 670 may be secured or securable to the second end region 627 according to any aspect of securement of the waist straps 160, 260, 360, 560, the one or more thigh straps 170, 270, or 470, and/or the one or more buttocks straps 570 to the fluid impermeable barriers 102, 202, 302, 402, 502 described above. For example, the buttock strap 670 may be fixedly or releasably secured to the fluid impermeable barrier 602 with one or more of an adhesive, molding, a mechanical attachment, a strip, an eyelet, ultrasonic welding, or any combination thereof. The buttock strap 670 may be secured or securable to the waist strap 660 with a threaded seam, ultrasonic welding, clips, hook and loop fasteners, buttons, snaps, a zipper, magnets, clasps or any other fastener. In some embodiments, the buttock strap is disposable and detachably secured or securable to the fluid collection device 600 and the waist strap 660 such that if a buttock strap 670 breaks or becomes soiled before the fluid collection device 600, the buttock strap 670 may be replaced with a new buttock strap 670.

In use, the legs of the user 150 may be inserted through the loop defined at least partially by the waist strap 660 and the loops at least partially defined by the buttock strap 670. The fluid collection assembly 601 may then be moved to position the fluid permeable body 120 of the fluid collection device 600 at least proximate (*e.g.,* adjacent) to the urethra of the user 150 with the waist strap 660 positioned around the waist of the user 150 and the buttock strap 670 positioned at least partially between the buttocks of the user 150. The waist strap 660 and/or the buttock strap 670 provide the technical effect of retaining the fluid collection device 600 in place with the fluid permeable body 120 positioned proximate to the urethra of the user 150 through the opening in the fluid impermeable barrier 602.

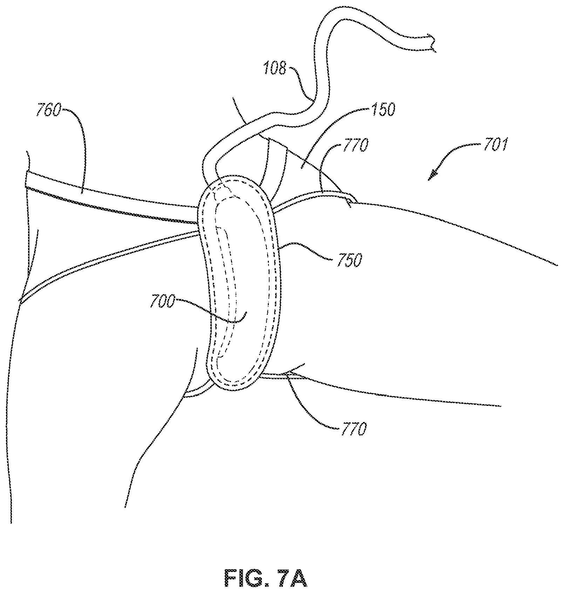

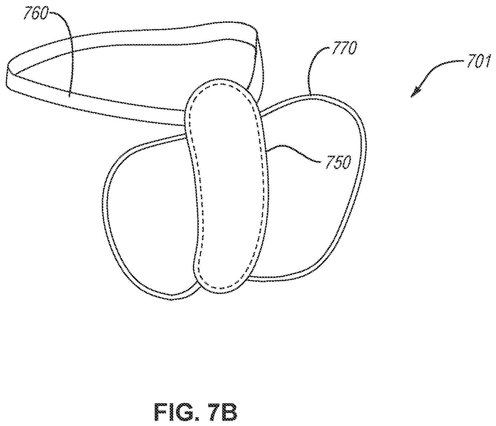

**FIG. 7A** is an isometric view of a fluid collection system 701 worn by the user 150, and **FIG. 7B** is an isometric view of the fluid collection system 701 without the user 150, according to an embodiment. The fluid collection system 701 includes a fluid collection device 700, a waist strap 760, and one or more thigh straps 770. Unless otherwise specified, the fluid collection device 700 may include any aspect of the fluid collection devices 100, 200, 300, 400, 500, 600 described above. For example, the fluid collection device 700 includes a fluid impermeable barrier having a first end region and a second end region distal to the first end region, with the fluid impermeable barrier defining an opening between the first end region and the second end region. The fluid collection device 700 also includes the fluid permeable body 120 positioned within a chamber defined by the fluid impermeable barrier. The waist strap 760 may include any aspect of the waist straps 160, 260, 360, 560, 660, and the one or more thigh straps 770 may include any aspect of the one or more thigh straps 170, 270, 470. Although shown with one or more thigh straps 770, in some embodiments, the one or more thigh straps 770 are absent, and the fluid collection system 701 includes one or more buttocks straps similar to those described above in relation to the fluid collection system 501, 601.

The fluid collection system 701 also includes a patch 750 providing the technical effect of retaining the fluid collection device 700 in a desired position. The patch may include any of a variety of materials, including a fabric material, a wicking material, a foam material, or combinations thereof. The waist strap 760 and the one or more thigh straps 770 are secured or securable to the patch 750. One or more buttocks straps also may be secured or securable to the patch 750. For example, the waist strap 760 and the one or more thigh straps 770 may secured or securable to the patch with one or more of a threaded seam, ultrasonic welding, an adhesive, clips, hook and loop fasteners, buttons, snaps, a zipper, magnets, clasps ultrasonic welding, or any other fastener. The waist strap 760 may be secured or securable to a first (or top) end region of the patch 750, the second (or bottom) end region of the patch 750, or between the first end region of the patch 750 and the second end region of the patch 750. The one or more thigh straps 770 may be secured or securable to the patch 750 at both the second end region of the patch and the first end region of the patch. The one or more thigh straps 770 may be secured or securable to the patch 750 at both the second end region of the patch and between the first end region of the patch and the second end region of the patch 750. In various embodiments, the fluid collection system 701 may include any configuration of straps described in relation to the fluid collection systems 101, 201, 301, 401, 501, 601 with the straps secured or securable to the patch 750 rather than the fluid collection device or conduit. At least one (*e.g.,* both) of the one or more thigh straps 770 and the waist strap 760 may be detachably secured or securable to the fluid collection device 700 such that the fluid collection device 700 may be used with different thigh straps 770 and/or waist straps 760, such as different sizes of thigh straps 770 and/or waist straps 760. Moreover, if at least one of the thigh straps 770 or the waist strap 760 becomes defective or soiled, the at least one of the thigh straps 770 or the waist strap 760 may be replaced with a new thigh straps 770 or waist strap 760 without disposing of the fluid collection device 700.

In use, the fluid collection assembly 701 may be used with any fluid collection device to provide the technical effect of retaining the fluid collection device in a desired position relative to the urethra of the user. For example, the legs of the user 150 may be inserted through the loop defined at least partially by the waist strap 760 and the loops at least partially defined by the one or more thigh straps 770. A fluid collection device 700 may be positioned proximate to the urethra of the user 150, and the fluid collection assembly 701 may be moved position the patch over the fluid collection device 700 and retain the fluid collection device 700 at least proximate to the urethra of the user 150. The waist strap 760, the one or more thigh straps, and the patch 750 provide the technical effect of retaining the fluid collection device 700 in place with the fluid permeable body 120 positioned proximate to the urethra of the user 150.

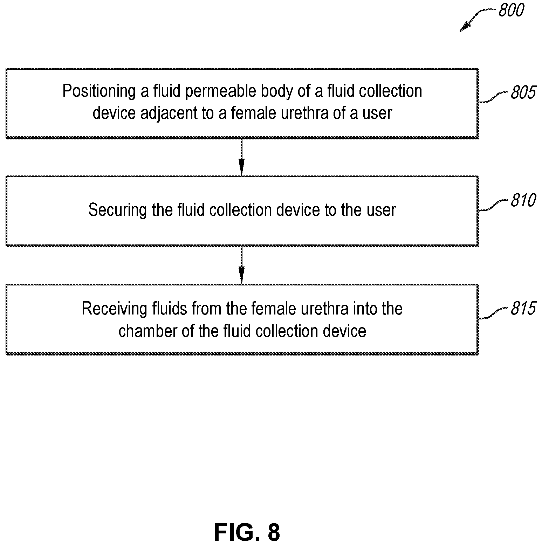

**FIG. 8** is a flow diagram of a method 800 for collecting fluids. The method 800 includes an act 805 of positioning a fluid permeable body of a fluid collection device adjacent to a female urethra of a user. The fluid permeable body is disposed within a chamber of a fluid impermeable barrier of the fluid collection device and exposed to the female urethra of the user through an opening in the fluid collection device defined by the fluid impermeable barrier.

The method 800 also includes an act 810 of securing the fluid collection device to the user. The act 810 securing the fluid collection device to the user may include positioning one or more straps around the user. Positioning the one or more straps around the user may include positioning at least a waist strap around a waist or hips of the user. In some embodiments, the method also may include fastening two ends of the waist strap together. In some embodiments, positioning the one or more straps around the user may include positioning a buttock strap between buttocks of the user, the buttock strap being secured to the fluid collection device and the waist strap. In some embodiments, positioning the one or more straps around the user may include positioning two buttocks straps over buttocks of the user, the two buttocks straps being secured to the fluid collection device and the waist strap. In some embodiments, positioning the one or more straps around the user may include positioning at least one or more thigh straps around thighs of the user. Positioning at least one or more thigh straps around thighs of the user may include positioning a single thigh strap around the thighs of the user. The method 800 also may include an act of fastening two ends of the single thigh strap together. In some embodiments, positioning at least one or more thigh straps around thighs of the user includes positioning two thigh straps around the thighs of the user. The method 800 also may include an act of fastening two ends of each thigh strap together. In some embodiments, the method 800 also may include an act of securing the one or more straps to the fluid collection device.

The method 800 also includes an act 815 of receiving fluids from the female urethra into the chamber of the fluid collection device. In some embodiments, the method 800 an act of applying suction effective to suction the fluids from the chamber via a conduit disposed therein.

Acts 805, 810, and 815 of the method 800 are for illustrative purposes. For example, the act 805, 810, and 815 of the method 800 may be performed in different orders, split into multiple acts, modified, supplemented, or combined. In an embodiment, one or more of the acts 805, 810, and 815 of the method 800 may be omitted from the method 800. Any of the acts 805, 810, and 815 may include using any of the fluid collection devices or systems disclosed herein.

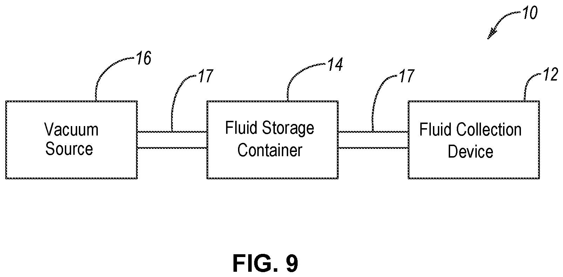

**FIG. 9** is a block diagram of a system 10 for fluid collection, according to an embodiment. The system 10 includes a fluid collection device 12, a fluid storage container 14, and a vacuum source 16 (*e.g.,* a portable vacuum source). The fluid collection device 12 may include any of the fluid collection devices described herein, such as the fluid collection devices 100, 200, 300, 400, 500, 600, 700. The fluid collection device 12, the fluid storage container 14, and the portable vacuum source 16 may be fluidly coupled to each other via one or more conduits 17. The conduit 17 may include any of the conduits described herein, such as the conduit 108. The fluid collection device 12 may be operably coupled to one or more of the fluid storage container 14 or the portable vacuum source via the conduit 17. Fluid (*e.g.,* urine or other bodily fluids) collected in the fluid collection device 12 may be removed from the fluid collection device 12 via the conduit 17, which protrudes into an interior region of the fluid collection device 12. For example, a first open end of the conduit 17 may extend into the fluid collection device 12 to a reservoir therein. The second open end of the conduit 17 may extend into the fluid storage container 14 or the portable vacuum source 16. The suction force may be introduced into the interior region of the fluid collection device 12 via the first open end of the conduit 17 responsive to a suction (*e.g.,* vacuum) force applied at the second end of the conduit 17. The suction force may be applied to the second open end of the conduit 17 by the portable vacuum source 16 either directly or indirectly.

The suction force may be applied indirectly via the fluid storage container 14. For example, the second open end of the conduit 17 may be disposed within the fluid storage container 14 and an additional conduit 17 may extend from the fluid storage container 14 to the portable vacuum source 16. Accordingly, the portable vacuum source 16 may apply suction to the fluid collection device 12 via the fluid storage container 14. The suction force may be applied directly via the fluid storage container 14. For example, the second open end of the conduit 17 may be disposed within the portable vacuum source 16. An additional conduit 17 may extend from the portable vacuum source 16 to a point outside of the fluid collection device 12, such as to the fluid storage container 14. In such examples, the portable vacuum source 16 may be disposed between the fluid collection device 12 and the fluid storage container 14.

The fluid collection device 12 may be shaped and sized to be positioned adjacent to a female urethra. The fluid collection member of the fluid collection device 12 may include a fluid impermeable barrier at least partially defining a chamber (*e.g.,* interior region of the fluid collection device member) of the fluid collection device 12. As described in more detail above, the fluid collection device 12 may include a softer, thinner fluid impermeable barrier than conventional fluid collection devices. The fluid impermeable barrier also defines an opening extending therethrough from the external environment. The opening may be positioned on the fluid collection member to be aligned adjacent to a female urethra. The fluid collection member of the fluid collection device 12 may include a fluid permeable body disposed within the fluid impermeable barrier. The fluid permeable body may include a fluid permeable membrane and fluid permeable support disposed within the fluid permeable membrane. The conduit 17 may extend into the fluid collection device 12 at a first end region, through one or more of the fluid impermeable barrier, fluid permeable membrane, or the fluid permeable support to a second end region of the fluid collection member of the fluid collection device 12. Example fluid collection devices for use with the systems and methods herein are described in more detail below.

In some embodiments, the fluid storage container 14 may include a bag (*e.g.,* drainage bag), a bottle or cup (*e.g.,* collection jar), or any other enclosed container for storing bodily fluids such as urine. In examples, the conduit 17 may extend from the fluid collection device 12 and attach to the fluid storage container 14 at a first point therein. An additional conduit 17 may attach to the fluid storage container 14 at a second point thereon and may extend and attach to the portable vacuum source 16. For example, the fluid storage container 14 may include a container fluidly coupled to a first conduit section that is also fluidly coupled to the fluid collection member of the fluid collection device 12. The container may be fluidly coupled to a second section of the conduit 17 that is also fluidly coupled to a portable vacuum source. In such examples, the portable vacuum source 16 may provide a vacuum/suction through the container to the fluid collection member to provide suction in the chamber of the fluid collection member. Accordingly, a vacuum (*e.g.,* suction) may be drawn through fluid collection device 12 via the fluid storage container 14. As the fluid is drained from the chamber, the fluid may travel through the first section of conduit to the fluid storage container where it may be retained. Fluid, such as urine, may be drained from the fluid collection device 12 using the portable vacuum source 16.

In some embodiments, the portable vacuum source 16 may be disposed in or on the fluid collection device 12. In such examples, the conduit 17 may extend from the fluid collection device and attach to the portable vacuum source 16 at a first point therein. An additional conduit 17 may attach to the portable vacuum source 16 at a second point thereon and may extend out of the fluid collection device 12, and may attach to the fluid storage container 14. Accordingly, a vacuum (*e.g.,* suction) may be drawn through fluid collection device 12 via the fluid storage container 14.

The portable vacuum source 16 may include one or more of a manual vacuum pump, and electric vacuum pump, a diaphragm pump, a centrifugal pump, a displacement pump, a magnetically driven pump, a peristaltic pump, or any pump configured to produce a vacuum. The portable vacuum source 16 may provide a vacuum or suction to remove fluid from the fluid collection member of the fluid collection device 12. In some embodiments, the portable vacuum source 16 may be powered by one or more of a power cord (*e.g.,* connected to a power socket), one or more batteries, or even manual power (*e.g.,* a hand operated vacuum pump). In examples, the portable vacuum source 16 may be sized and shaped to fit outside of, on, or within the fluid collection device 12. For example, the portable vacuum source 16 may include one or more miniaturized pumps or one or more micro pumps. The portable vacuum sources 16 disclosed herein may include one or more of a switch, a button, a plug, a remote, or any other device suitable to activate the portable vacuum source 16. It should be understood that the portable vacuum sources 16 disclosed herein may provide a portable means of providing a suction or vacuum that allows use of the devices and systems herein outside of hospital or care facility environments where vacuum lines are plumbed into patient rooms or large (*e.g.,* larger or heavier than a patient can readily carry) vacuum sources are located. For example, a portable vacuum source may be small and light enough to be carried by a user (*e.g.,* patient) or aid (*e.g.,* nurse) during transportation of the user. In some embodiments, the vacuum source 16 is not portable, and may include a wall-mounted vacuum source.

As used herein, the term "about" or "substantially" refers to an allowable variance of the term modified by "about" by ±10% or ±5%. Further, the terms "less than," "or less," "greater than", "more than," or "or more" include as an endpoint, the value that is modified by the terms "less than," "or less," "greater than," "more than," or "or more."

While various aspects and embodiments have been disclosed herein, other aspects and embodiments are contemplated. The various aspects and embodiment disclosed herein are for purposes of illustration and are not intended to be limiting.

## Claims

1. A fluid collection system, comprising:
a fluid collection device (100, 200, 300, 400, 500, 600) including:
a fluid impermeable barrier (102, 202, 302, 402, 502, 602) at least partially defining a chamber (204), an opening (106, 206, 306, 406) extending longitudinally along the fluid impermeable barrier and configured to be positioned adjacent to a urethra of a user, and an aperture configured to receive a conduit (108) therethrough; and
a fluid permeable body (120) positioned at least partially within the chamber to extend across at least a portion of the opening and configured to wick fluid away from the opening; and
one or more straps (160, 170, 260, 270, 360, 470, 560, 570, 660) configured to wrap around the user to secure the fluid collection device adjacent to the urethra of the user,
**characterised in that**
at least one strap of the one or more straps is fixedly or releasably secured or securable to the fluid impermeable barrier.

2. The fluid collection system of claim 1, wherein the one or more straps include at least a waist strap (160, 260, 360, 560, 660) secured to the fluid impermeable barrier and configured to wrap around a waist of the user.

3. The fluid collection system of claim 2, wherein the waist strap (160, 260, 560, 660) is secured or securable to the fluid impermeable barrier at a first end region of the fluid collection device proximate to the aperture.

4. The fluid collection system of claim 2, wherein the waist strap (360) is secured or securable to the fluid collection device opposite to a portion of the opening.

5. The fluid collection system of any of claims 2-4, wherein the waist strap is releasably secured or securable to the fluid collection device.

6. The fluid collection system of any of claims 2-4, wherein the waist strap is fixedly secured to the fluid collection device.

7. The fluid collection system of any of claims 2-6, wherein the fluid impermeable barrier includes a second end region distal to the aperture and the fluid collection device includes one or more buttocks straps (570, 670) secured or securable to the second end region of the fluid collection device and the waist strap.

8. The fluid collection system of claim 7, wherein each of the one or more buttocks straps is fixedly secured to at least one of the fluid collection device or the waist strap.

9. The fluid collection system of any of claims 1-8, wherein the one or more straps include at least one or more thigh straps (170, 270, 470).

10. The fluid collection system of claim 9, wherein the one or more thigh straps include a single thigh strap configured to wrap around the thighs of the user.

11. The fluid collection system of claim 10, wherein the single thigh strap is secured or securable to the fluid impermeable barrier at a first end region of the fluid collection device proximate to the aperture; and optionally wherein the single thigh strap is secured or securable to the fluid collection device opposite to a portion of the opening.

12. The fluid collection system of any of claims 10-11, wherein the single thigh strap is releasably secured or securable to the fluid collection device.

13. The fluid collection system of any of claims 10-11, wherein the single thigh strap is fixedly secured to the fluid collection device.

14. The fluid collection system of claim 9, wherein the one or more thigh straps include two thigh straps each configured to wrap a different thigh of the user, wherein the two thigh straps are each secured or securable to the fluid impermeable barrier at a first end region of the fluid collection device proximate to the aperture and also secured or securable to the fluid impermeable barrier at a second end region of the fluid collection device distal to the first end region, and wherein the one or more straps include a waist strap secured to the fluid impermeable barrier and configured to wrap around a waist of the user, the waist strap being secured or securable to the fluid impermeable barrier at the first end region of the fluid collection device proximate to the aperture.

15. The fluid collection system of claim 1, wherein the at least one strap is fixedly or releasably secured or securable to the fluid impermeable barrier with one or more of an adhesive, a molding, a strip on the fluid impermeable barrier, or an eyelet on the fluid impermeable barrier.

## Patentansprüche

1. Fluidsammelsystem, umfassend:
eine Fluidsammelvorrichtung (100, 200, 300, 400, 500, 600) einschließlich:
einer fluidundurchlässigen Barriere (102, 202, 302, 402, 502, 602), die zumindest teilweise eine Kammer (204), eine Öffnung (106, 206, 306, 406), die sich in Längsrichtung entlang der fluidundurchlässigen Barriere erstreckt und so konfiguriert ist, dass sie angrenzend an eine Harnröhre eines Benutzers positioniert wird, und eine Öffnung definiert, die so konfiguriert ist, dass sie einen Kanal (108) durch sie hindurch aufnimmt; und
eines fluiddurchlässigen Körpers (120), der zumindest teilweise innerhalb der Kammer positioniert ist, um sich über zumindest einen Abschnitt der Öffnung zu erstrecken, und so konfiguriert ist, dass er Fluid von der Öffnung abtransportiert; und
eines oder mehrerer Riemen (160, 170, 260, 270, 360, 470, 560, 570, 660), die so konfiguriert sind, dass sie sich um den Benutzer wickeln, um die Fluidsammelvorrichtung an die Harnröhre des Benutzers angrenzend zu befestigen, **dadurch gekennzeichnet, dass**
mindestens ein Riemen des einen oder der mehreren Riemen fest oder lösbar an der fluidundurchlässigen Barriere befestigt oder befestigbar ist.

2. Fluidsammelsystem nach Anspruch 1, wobei der eine oder die mehreren Riemen mindestens einen Taillenriemen (160, 260, 360, 560, 660) einschließen, der an der fluidundurchlässigen Barriere befestigt und so konfiguriert ist, dass er sich um die Taille des Benutzers wickelt.

3. Fluidsammelsystem nach Anspruch 2, wobei der Taillenriemen (160, 260, 560, 660) an der fluidundurchlässigen Barriere an einem ersten Endbereich der Fluidsammelvorrichtung in der Nähe der Öffnung befestigt oder befestigbar ist.

4. Fluidsammelsystem nach Anspruch 2, wobei der Taillenriemen (360) gegenüber einem Abschnitt der Öffnung an der Fluidsammelvorrichtung befestigt oder befestigbar ist.

5. Fluidsammelsystem nach einem der Ansprüche 2-4, wobei der Taillenriemen lösbar an der Fluidsammelvorrichtung befestigt oder befestigbar ist.

6. Fluidsammelsystem nach einem der Ansprüche 2-4, wobei der Taillenriemen fest an der Fluidsammelvorrichtung befestigt ist.

7. Fluidsammelsystem nach einem der Ansprüche 2-6, wobei die fluidundurchlässige Barriere einen zweiten Endbereich distal zur Öffnung einschließt und die Fluidsammelvorrichtung einen oder mehrere Gesäßriemen (570, 670) einschließt, die an dem zweiten Endbereich der Fluidsammelvorrichtung und dem Taillenriemen befestigt oder befestigbar sind.

8. Fluidsammelsystem nach Anspruch 7, wobei jeder des einen oder der mehreren Gesäßriemen fest an mindestens einem von der Fluidsammelvorrichtung oder dem Taillenriemen befestigt ist.

9. Fluidsammelsystem nach einem der Ansprüche 1-8, wobei der eine oder die mehreren Riemen mindestens einen oder mehrere Oberschenkelriemen (170, 270, 470) einschließen.

10. Fluidsammelsystem nach Anspruch 9, wobei der eine oder die mehreren Oberschenkelriemen einen einzelnen Oberschenkelriemen einschließen, der so konfiguriert ist, dass er sich um die Oberschenkel des Benutzers wickelt.

11. Fluidsammelsystem nach Anspruch 10, wobei der einzelne Oberschenkelriemen an der fluidundurchlässigen Barriere an einem ersten Endbereich der Fluidsammelvorrichtung in der Nähe der Öffnung befestigt oder befestigbar ist; und optional wobei der einzelne Oberschenkelriemen gegenüber einem Abschnitt der Öffnung an der Fluidsammelvorrichtung befestigt oder befestigbar ist.

12. Fluidsammelsystem nach einem der Ansprüche 10-11, wobei der einzelne Oberschenkelriemen lösbar an der Fluidsammelvorrichtung befestigt oder befestigbar ist.

13. Fluidsammelsystem nach einem der Ansprüche 10-11, wobei der einzelne Oberschenkelriemen fest an der Fluidsammelvorrichtung befestigt ist.

14. Fluidsammelsystem nach Anspruch 9, wobei der eine oder die mehreren Oberschenkelriemen zwei Oberschenkelriemen einschließen, die jeweils so konfiguriert sind, dass sie sich um einen anderen Oberschenkel des Benutzers wickeln, wobei die beiden Oberschenkelriemen jeweils an der fluidundurchlässigen Barriere an einem ersten Endbereich der Fluidsammelvorrichtung in der Nähe der Öffnung befestigt oder befestigbar sind und ebenfalls an der fluidundurchlässigen Barriere an einem zweiten Endbereich der Fluidsammelvorrichtung distal zum ersten Endbereich befestigt oder befestigbar sind, und wobei der eine oder die mehreren Riemen einen Taillenriemen einschließen, der an der fluidundurchlässigen Barriere befestigt und so konfiguriert ist, dass er sich um die Taille des Benutzers wickelt, wobei der Taillenriemen an der fluidundurchlässigen Barriere am ersten Endbereich der Fluidsammelvorrichtung in der Nähe der Öffnung befestigt oder befestigbar ist.

15. Fluidsammelsystem nach Anspruch 1, wobei der mindestens eine Riemen fest oder lösbar an der fluidundurchlässigen Barriere mit einem oder mehreren von einem Klebstoff, einem Formteil, einem Streifen an der fluidundurchlässigen Barriere oder einer Öse an der fluidundurchlässigen Barriere befestigt oder befestigbar ist.

## Revendications

1. Système de collecte de fluide, comprenant :
un dispositif (100, 200, 300, 400, 500, 600) de collecte de fluide incluant :
une barrière (102, 202, 302, 402, 502, 602) imperméable au fluide, définissant au moins partiellement une chambre (204), une ouverture (106, 206, 306, 406) s'étendant longitudinalement le long de la barrière imperméable au fluide et configurée pour être positionnée de façon adjacente à un urètre d'un utilisateur, et une ouverture configurée pour recevoir un conduit (108) à travers elle ; et
un corps (120) perméable au fluide, positionné au moins partiellement à l'intérieur de la chambre de façon à s'étendre sur au moins une partie de l'ouverture et configuré pour évacuer le fluide à partir de l'ouverture ; et
une ou plusieurs sangles (160, 170, 260, 270, 360, 470, 560, 570, 660) configurées pour s'enrouler autour de l'utilisateur pour fixer le dispositif de collecte de fluide de façon adjacente à l'urètre de l'utilisateur, **caractérisé en ce que**
au moins une sangle parmi la ou les sangles est fixée ou peut être fixée de manière solidaire ou amovible à la barrière imperméable au fluide.

2. Système de collecte de fluide selon la revendication 1, dans lequel la sangle ou les sangles incluent au moins une sangle de taille (160, 260, 360, 560, 660) fixée à la barrière imperméable au fluide et configurée pour s'enrouler autour d'une taille de l'utilisateur.

3. Système de collecte de fluide selon la revendication 2, dans lequel la sangle de taille (160, 260, 560, 660) est fixée ou peut être fixée à la barrière imperméable au fluide au niveau d'une première région d'extrémité du dispositif de collecte de fluide à proximité de l'ouverture.

4. Système de collecte de fluide selon la revendication 2, dans lequel la sangle de taille (360) est fixée ou peut être fixée au dispositif de collecte de fluide opposé à une partie de l'ouverture.

5. Système de collecte de fluide selon l'une quelconque des revendications 2 à 4, dans lequel la sangle de taille est fixée ou peut être fixée au dispositif de collecte de fluide.

6. Système de collecte de fluide selon l'une quelconque des revendications 2 à 4, dans lequel la sangle de taille est fixée de manière solidaire au dispositif de collecte de fluide.

7. Système de collecte de fluide selon l'une quelconque des revendications 2 à 6, dans lequel la barrière imperméable au fluide inclut une seconde région d'extrémité distale à l'ouverture et le dispositif de collecte de fluide inclut une ou plusieurs sangles de fesse (570, 670) fixées ou pouvant être fixées à la seconde région d'extrémité du dispositif de collecte de fluide et de la sangle de taille.

8. Système de collecte de fluide selon la revendication 7, dans lequel chacune de la ou des sangles de fesse est fixée de manière solidaire à au moins l'un(e) du dispositif de collecte de fluide ou de la sangle de taille.

9. Système de collecte de fluide selon l'une quelconque des revendications 1 à 8, dans lequel la sangle ou les sangles incluent au moins une ou plusieurs sangles de cuisse (170, 270, 470).

10. Système de collecte de fluide selon la revendication 9, dans lequel la sangle ou les sangles incluent une unique sangle de cuisse configurée pour s'enrouler autour des cuisses de l'utilisateur.

11. Système de collecte de fluide selon la revendication 10, dans lequel l'unique sangle de cuisse est fixée ou peut être fixée à la barrière imperméable au fluide au niveau d'une première région d'extrémité du dispositif de collecte de fluide à proximité de l'ouverture ; et éventuellement dans lequel l'unique sangle de cuisse est fixée ou peut être fixée au dispositif de collecte de fluide opposé à une partie de l'ouverture.

12. Système de collecte de fluide selon l'une quelconque des revendications 10 à 11, dans lequel l'unique sangle de cuisse est fixée ou peut être fixée de manière amovible au dispositif de collecte de fluide.

13. Système de collecte de fluide selon l'une quelconque des revendications 10 à 11, dans lequel l'unique sangle de cuisse est fixée de manière solidaire au dispositif de collecte de fluide.

14. Système de collecte de fluide selon la revendication 9, dans lequel la ou les sangles de cuisse incluent deux sangles de cuisse, chacune configurées pour s'enrouler autour d'une cuisse différente de l'utilisateur, dans lequel les deux sangles de cuisse sont chacune fixées ou peuvent chacune être fixées à la barrière imperméable au fluide au niveau d'une première région d'extrémité du dispositif de collecte de fluide à proximité de l'ouverture et sont également fixées ou peuvent être fixées à la barrière imperméable au fluide au niveau d'une seconde région d'extrémité du dispositif de collecte de fluide distale à la première région d'extrémité, et dans lequel la sangle ou les sangles incluent une sangle de taille fixée à la barrière imperméable au fluide et est/sont configurée(s) pour s'enrouler autour d'une taille de l'utilisateur, la sangle de taille étant fixée ou pouvant être fixée à la barrière imperméable au fluide au niveau de la première région d'extrémité du dispositif de collecte de fluide à proximité de l'ouverture.

15. Système de collecte de fluide selon la revendication 1, dans lequel au moins une sangle de taille est fixée ou peut être fixée à la barrière imperméable au fluide avec un ou plusieurs d'un adhésif, d'un moulage, d'une bande sur la barrière imperméable au fluide, ou d'un oeillet sur la barrière imperméable au fluide.
